# EUROPEAN PATENT APPLICATION

(11) **EP 3 205 356 A1**
(43) Date of publication of application: **16.08.2017**
(21) Application number: 16155003.3
(22) Date of filing: 10.02.2016
(51) Int. Cl.: A61K 48/00, C12N 15/85

(54) **PLASMID EXPRESSION VECTOR ENCODING HUMAN INTERLEUKIN 12 UNDER TRANSCRIPTIONAL CONTROL OF P21 PROMOTER AND WITHOUT ANTIBIOTIC RESISTANCE FOR CANCER GENE THERAPY AND OTHER USES THEREOF**

(71) Applicant: Onkoloski Institut Ljubljana, 1000 Ljubljana (SI); Univerza na Primorskem, Universita' del Litorale, 6000 Koper (SI)
(72) Inventor: Cemazar, Prof. Dr. Maja, 1000 Ljubljana (SI); Sersa, Prof. Dr. Gregor, 1000 Ljubljana (SI); Kamensek, Dr. Urska, 1000 Ljubljana (SI); Tesic, Dr. Natasa, 6310 Izola (SI)
(74) Representative: Marton, Dan-Robert

(57) **Abstract**

The present invention relates to an expression plasmid comprising a polynucleotide encoding an IL-12 (Interleukin 12) polypeptide and operably linked thereto a CDKN1A promoter, wherein said expression plasmid does not comprise a selectable marker gene.

The expression plasmid can be used for the treatment of cancer.

SEQUENCE LISTING

<110>  Institute of Oncology and University of Primorska

<120>  Plasmid expression vector encoding human interleukin 12 under
       transcriptional control of p21 promoter and without antibiotic
       resistance for cancer gene therapy and other uses thereof

<130>  Human IL-12

<160>  7

<170>  PatentIn version 3.3

<210>  1
<211>  6054
<212>  DNA
<213>  artificial

<220>
<223>  Plasmid vector

<400>  1
aagggcgaat tctgcagata tccatcacac tggcggccgc tcgagctcaa gcttcgaatt      60

cgatatcaag cttgggcagc aggctgtggc tctgattggc tttctggcca ttaggaacat     120

gtcccaacat gttgagctct ggcatagaag aggctggtgg ctattttgtc cttgggctgc     180

ctgttatcag gtgaggaagg ggatggtagg agacagaaga cctctaaagg ccccaggtaa     240

gccttagcct gttactctga acagggtatg tgatctgcca gcagatcctt gcgacagggc     300

tgggatctga tgcatgtgtg cttgtgtgag tgtgtgctgg gagtcagatt ctgtgtgtga     360

cttttaacag cctgctccct tgccttcctc agggcagaag tcctccctta gagtgtgtct     420

gggtacacat tcaagtgcat ggttgcaaac tttttttttt aaagcactga atagtactag     480

acacttagta ggtacttaag aaatattgaa tgtcgtggtg gtggtgagct agaagttata     540

aaaaaaattc tttcccaaaa acaacaacaa aaagaattat ttcattgtga agctcagtac     600

cacaaaaatt taaataattc attacaagcc ttcattaaaa aaaaatttct ccccgaagta     660

aacagacaga caatgtctag tctatttgaa atgcctgaaa gcagaggggc ttcaaggcag     720

tgggagaagg tgcctgtcct ctgctggaca tttgacaacc agccctttgg atggcttgta     780

tgtataggag cgaaggtgca gacagcagtg gggcttagag tggggtcctg aggctgtgct     840

gtggcccttc tggggtttag ccacaatcct ggcctgactc cagggcgagg caggccaagg     900

gggcctgctg ctgtgtcctc ccacccctac ctgggctccc atccccacag cagaggagaa     960

agaagcctgt cctccccgag gtcagctgcg ttagaggaag aagactgggc atgtctgggc    1020

agagatttcc agactctgag cagcctgaga tgtcagtaat tgtagctgct ccaagcctgg    1080

gttctgtttt tcagtgggat ttctgttcag atgaacaatc catcctctgc aattttttaa    1140

aagcaaaact gcaaatgttt caggcacaga aaggaggcaa aggtgaagtc caggggaggt    1200

caggggtgtg aggtagatgg gagcggatag gcacatcact catttctgtg tctgtcagaa 1260
gaaccagtag acacttccag aattgtcctt tatttacgtc atctccataa accatctgca 1320
aatgagggtt atttggcatt tttgtcattt tggaaccaca gaaataaagg atgacaagca 1380
gagagccccg ggcaggaggc aaaagtcctg tgttccaact atagtcattt ctttgctgca 1440
tgatctgagt taggtcacca gacttctctg agccccagtt tccccagcag tgtatacggg 1500
ctatgtgggg agtattcagg agacagacaa ctcactcgtc aaatcctccc cttcctggcc 1560
aacaaagctg ctgcaaccac aggggtttct tctgttcagg tgagtgtagg gtgtagggag 1620
attggttcaa tgtccaattc ttctgtttcc ctggagatca ggttgccctt ttttggtagt 1680
ctctccaatt ccctccttcc cggaagcatg tgacaatcaa caactttgta tacttaagtt 1740
cagtggacct caatttcctc atctgtgaaa taaacgggac tgaaaaatca ttccggcctc 1800
aagatgcttt gttggggtgt ctaggtgctc caggtgcttc tgggagaggt gacctagtga 1860
gggatcagtg ggaatagagg tgatattgtg gggcttttct ggaaattgca gagaggtgca 1920
tcgtttttat aatttatgaa tttttatgta ttaatgtcat cctcctgatc ttttcagctg 1980
cattgggtaa atccttgcct gccagagtgg gtcagcggtg agccagaaag ggggctcatt 2040
ctaacagtgc tgtgtcctcc tggagagtgc caactcattc tccaagtaaa aaaagccaga 2100
tttgtggctc acttcgtggg gaaatgtgtc cagcgcacca acgcaggcga gggactgggg 2160
gaggagggaa gtgcccctct gcagcacgcg aggttccggg accggctggc ctgctggaac 2220
tcggccaggc tcagctggct ccgcgctggg cagccaggag cctgggcccc ggggagggcg 2280
gtcccgggcg gcgcggtggg ccgagcgcgg gtcccgcctc cttgaggcgg gcccgggcgg 2340
ggcggttgta tatcagggcc gcgcagagct gcgccagctg aggtgtgagc agctgccgaa 2400
gtcagaagct tatcgatacc gtcgactact aaccttcttc tctttcctac agctgagatc 2460
accggcgaag gagggccacc atgggtcacc agcagttggt catctcttgg ttttccctgg 2520
tttttctggc atctcccctc gtggccatat gggaactgaa gaaagatgtt tatgtcgtag 2580
aattggattg gtatccggat gcccctggag aaatggtggt cctcacctgt gacacccctg 2640
aagaagatgg tatcacctgg accttggacc agagcagtga ggtcttaggc tctggcaaaa 2700
ccctgaccat ccaagtcaaa gagtttggag atgctggcca gtacacctgt cacaaaggag 2760
gcgaggttct aagccattcg ctcctgctgc ttcacaaaaa ggaagatgga atttggtcca 2820
ctgatatttt aaaggaccag aaagaaccca aaaataagac ctttctaaga tgcgaggcca 2880
agaattattc tggacgtttc acctgctggt ggctgacgac aatcagtact gatttgacat 2940
tcagtgtcaa aagcagcaga ggctcttctg acccccaagg ggtgacgtgc ggagctgcta 3000
cactctctgc agagagagtc agaggggaca acaaggagta tgagtactca gtggagtgcc 3060
aggaggacag tgcctgccca gctgctgagg agagtctgcc cattgaggtc atggtggatg 3120

ccgttcacaa gctcaagtat gaaaactaca ccagcagctt cttcatcagg gacatcatca 3180

aacctgaccc acccaagaac ttgcagctga agccattaaa gaattctcgg caggtggagg 3240

tcagctggga gtaccctgac acctggagta ctccacattc ctacttctcc ctgacattct 3300

gcgttcaggt ccagggcaag agcaagagag aaaagaaaga tagagtcttc acggacaaga 3360

cctcagccac ggtcatctgc cgcaaaaatg ccagcattag cgtgcgggcc caggaccgct 3420

actatagctc atcttggagc gaatgggcat ctgtgccctg cagtgttcct ggagtagggg 3480

tacctggggt gggcgccaga aacctccccg tggccactcc agacccagga atgttcccat 3540

gccttcacca ctcccaaaac ctgctgaggg ccgtcagcaa catgctccag aaggccagac 3600

aaactctaga attttaccct tgcacttctg aagagattga tcatgaagat atcacaaaag 3660

ataaaaccag cacagtggag gcctgtttac cattggaatt aaccaagaat gagagttgcc 3720

taaattccag agagacctct ttcataacta atgggagttg cctggcctcc agaaagacct 3780

cttttatgat ggccctgtgc cttagtagta tttatgaaga cttgaagatg taccaggtgg 3840

agttcaagac catgaatgca aagctgctga tggatcctaa gaggcagatc tttctagatc 3900

aaaacatgct ggcagttatt gatgagctga tgcaggccct gaatttcaac agtgagactg 3960

tgccacaaaa atcctccctt gaagaaccgg atttttataa aactaaaatc aagctctgca 4020

tacttcttca tgctttcaga attcgggcag tgactattga tagagtgatg agctatctga 4080

atgcttccta aaaagcgagg tccctccaaa ccgttgtcat ttttataaaa ctttgaaatg 4140

aggaaacttt gataggatgt ggattaagaa ctagggaggg gctagatcat aatcagccat 4200

accacatttg tagaggtttt acttgcttta aaaaacctcc cacacctccc cctgaacctg 4260

aaacataaaa tgaatgcaat tgttgttgtt aacttgttta ttgcagctta taatggttac 4320

aaataaagca atagcatcac aaatttcaca aataaagcat ttttttcact gcattctagt 4380

tgtggtttgt ccaaactcat caatgtatct taaggcgtaa attgtaagcg ttaatgtgct 4440

aaaacttcat ttttaattta aaaggatcta ggtgaagatc ctttttgata atctcatgac 4500

caaaatccct taacgtgagt tttcgttcca ctgagcgtca gaccccgtag aaaagatcaa 4560

aggatcttct tgagatcctt tttttctgcg cgtaatctgc tgcttgcaaa caaaaaaacc 4620

accgctacca gcggtggttt gtttgccgga tcaagagcta ccaactcttt ttccgaaggt 4680

aactggcttc agcagagcgc agataccaaa tactgttctt ctagtgtagc cgtagttagg 4740

ccaccacttc aagaactctg tagcaccgcc tacatacctc gctctgctaa tcctgttacc 4800

agtggctgct gccagtggcg ataagtcgtg tcttaccggg ttggactcaa gacgatagtt 4860

accggataag gcgcagcggt cgggctgaac ggggggttcg tgcacacagc ccagcttgga 4920

gcgaacgacc tacaccgaac tgagatacct acagcgtgag ctatgagaaa gcgccacgct 4980

tcccgaaggg agaaaggcgg acaggtatcc ggtaagcggc agggtcggaa caggagagcg 5040

cacgagggag cttccagggg gaaacgcctg gtatctttat agtcctgtcg ggtttcgcca 5100

cctctgactt gagcgtcgat ttttgtgatg ctcgtcaggg gggcggagcc tatggaaaaa 5160

cgccagcaac gcggcctttt tacggttcct ggccttttgc tggccttttg ctcacatgtt 5220

ctttcctgcg ttatcccctg attctgtgga taaccgtatt accgcctttg agtgagctga 5280

taccgctcgc cgcagccgaa cgaccgagcg cagcgagtca gtgagcgagg aagcggaaga 5340

gcgcccaata cgcaaaccgc ctctccccgc gcgttggccg attcattaat gcagctggca 5400

cgacaggttt cccgactgga aagcgggcag tgagcgcaac gcaattaatg tgagttagct 5460

cactcattag gcaccccagg ctttacactt tatgcttccg gctcgtatgt tgtgtggaat 5520

tgtgagcgga taacaatttc acacaggaaa cagctatgac catgattacg ccaagctatt 5580

taggtgacac tatagaatac tcaagctatg catcaagctt ggtaccgagc tcggatccac 5640

tagtaacggc cgccagtgtg ctggaattcg cccttataat gctagcggtc atgaagctta 5700

tcatcgataa gctcatagac agcctgaaac aggcgatgct gcttatcgaa tcaaagctgc 5760

cgacaacacg ggagccagtg acgcctcccg tggggaaaaa atcatggcaa ttctggaaga 5820

aatagcgctt tcagccggca aaccggctga agccggatct gcgattctga taacaaacta 5880

gcaacaccag aacagcccgt ttgcgggcag caaaacccgt acttttggac gttccggcgg 5940

ttttttgtgg cgagtggtgt tcgggcggtg cgcgcaagat ccattatgtt aaacgggcga 6000

gtttacatct caaaaccgcc cgcttaacac cattcatgag gccgctagca ttat 6054

<210> 2
<211> 2380
<212> DNA
<213> Homo sapiens

<400> 2
ctcaagcttc gaattcgata tcaagcttgg gcagcaggct gtggctctga ttggctttct 60

ggccattagg aacatgtccc aacatgttga gctctggcat agaagaggct ggtggctatt 120

ttgtccttgg gctgcctgtt atcaggtgag gaaggggatg gtaggagaca gaagacctct 180

aaaggcccca ggtaagcctt agcctgttac tctgaacagg gtatgtgatc tgccagcaga 240

tccttgcgac agggctggga tctgatgcat gtgtgcttgt gtgagtgtgt gctgggagtc 300

agattctgtg tgtgactttt aacagcctgc tcccttgcct tcctcagggc agaagtcctc 360

ccttagagtg tgtctgggta cacattcaag tgcatggttg caaacttttt tttttaaagc 420

actgaatagt actagacact tagtaggtac ttaagaaata ttgaatgtcg tggtggtggt 480

gagctagaag ttataaaaaa aattctttcc caaaaacaac aacaaaaaga attatttcat 540

tgtgaagctc agtaccacaa aaatttaaat aattcattac aagccttcat taaaaaaaaa 600

tttctccccg aagtaaacag acagacaatg tctagtctat ttgaaatgcc tgaaagcaga 660

ggggcttcaa ggcagtggga gaaggtgcct gtcctctgct ggacatttga caaccagccc 720

tttggatggc ttgtatgtat aggagcgaag gtgcagacag cagtggggct tagagtgggg 780

tcctgaggct gtgctgtggc ccttctgggg tttagccaca atcctggcct gactccaggg 840

cgaggcaggc caagggggcc tgctgctgtg tcctcccacc cctacctggg ctcccatccc 900

cacagcagag gagaaagaag cctgtcctcc ccgaggtcag ctgcgttaga ggaagaagac 960

tgggcatgtc tgggcagaga tttccagact ctgagcagcc tgagatgtca gtaattgtag 1020

ctgctccaag cctgggttct gtttttcagt gggatttctg ttcagatgaa caatccatcc 1080

tctgcaattt tttaaaagca aaactgcaaa tgtttcaggc acagaaagga ggcaaaggtg 1140

aagtccaggg gaggtcaggg gtgtgaggta gatgggagcg gataggcaca tcactcattt 1200

ctgtgtctgt cagaagaacc agtagacact tccagaattg tcctttattt acgtcatctc 1260

cataaaccat ctgcaaatga gggttatttg gcatttttgt cattttggaa ccacagaaat 1320

aaaggatgac aagcagagag ccccgggcag gaggcaaaag tcctgtgttc caactatagt 1380

catttctttg ctgcatgatc tgagttaggt caccagactt ctctgagccc cagtttcccc 1440

agcagtgtat acgggctatg tggggagtat tcaggagaca gacaactcac tcgtcaaatc 1500

ctccccttcc tggccaacaa agctgctgca accacagggg tttcttctgt tcaggtgagt 1560

gtagggtgta gggagattgg ttcaatgtcc aattcttctg tttccctgga gatcaggttg 1620

cccttttttg gtagtctctc caattccctc cttcccggaa gcatgtgaca atcaacaact 1680

ttgtatactt aagttcagtg gacctcaatt tcctcatctg tgaaataaac gggactgaaa 1740

aatcattccg gcctcaagat gctttgttgg ggtgtctagg tgctccaggt gcttctggga 1800

gaggtgacct agtgagggat cagtgggaat agaggtgata ttgtggggct tttctggaaa 1860

ttgcagagag gtgcatcgtt tttataattt atgaattttt atgtattaat gtcatcctcc 1920

tgatcttttc agctgcattg ggtaaatcct tgcctgccag agtgggtcag cggtgagcca 1980

gaaagggggc tcattctaac agtgctgtgt cctcctggag agtgccaact cattctccaa 2040

gtaaaaaaag ccagatttgt ggctcacttc gtggggaaat gtgtccagcg caccaacgca 2100

ggcgagggac tgggggagga gggaagtgcc cctctgcagc acgcgaggtt ccgggaccgg 2160

ctggcctgct ggaactcggc caggctcagc tggctccgcg ctgggcagcc aggagcctgg 2220

gccccgggga gggcggtccc gggcggcgcg gtgggccgag cgcgggtccc gcctccttga 2280

ggcgggcccg ggcggggcgg ttgtatatca gggccgcgca gagctgcgcc agctgaggtg 2340

tgagcagctg ccgaagtcag aagcttatcg ataccgtcga 2380

<210> 3
<211> 1703

<212>  DNA
<213>  Homo sapiens

<400>  3
ccatgggtca ccagcagttg gtcatctctt ggttttccct ggtttttctg gcatctcccc      60

tcgtggccat atgggaactg aagaaagatg tttatgtcgt agaattggat tggtatccgg     120

atgcccctgg agaaatggtg gtcctcacct gtgacacccc tgaagaagat ggtatcacct     180

ggaccttgga ccagagcagt gaggtcttag gctctggcaa aaccctgacc atccaagtca     240

aagagtttgg agatgctggc cagtacacct gtcacaaagg aggcgaggtt ctaagccatt     300

cgctcctgct gcttcacaaa aaggaagatg gaatttggtc cactgatatt ttaaaggacc     360

agaaagaacc caaaaataag acctttctaa gatgcgaggc caagaattat tctggacgtt     420

tcacctgctg gtggctgacg acaatcagta ctgatttgac attcagtgtc aaaagcagca     480

gaggctcttc tgacccccaa ggggtgacgt gcggagctgc tacactctct gcagagagag     540

tcagagggga caacaaggag tatgagtact cagtggagtg ccaggaggac agtgcctgcc     600

cagctgctga ggagagtctg cccattgagg tcatggtgga tgccgttcac aagctcaagt     660

atgaaaacta caccagcagc ttcttcatca gggacatcat caaacctgac ccacccaaga     720

acttgcagct gaagccatta aagaattctc ggcaggtgga ggtcagctgg gagtaccctg     780

acacctggag tactccacat tcctacttct ccctgacatt ctgcgttcag gtccagggca     840

agagcaagag agaaaagaaa gatagagtct tcacggacaa gacctcagcc acggtcatct     900

gccgcaaaaa tgccagcatt agcgtgcggg cccaggaccg ctactatagc tcatcttgga     960

gcgaatgggc atctgtgccc tgcagtgttc ctggagtagg ggtacctggg gtgggcgcca    1020

gaaacctccc cgtggccact ccagacccag gaatgttccc atgccttcac cactcccaaa    1080

acctgctgag ggccgtcagc aacatgctcc agaaggccag acaaactcta gaattttacc    1140

cttgcacttc tgaagagatt gatcatgaag atatcacaaa agataaaacc agcacagtgg    1200

aggcctgttt accattggaa ttaaccaaga atgagagttg cctaaattcc agagagacct    1260

ctttcataac taatgggagt tgcctggcct ccagaaagac ctcttttatg atggccctgt    1320

gccttagtag tatttatgaa gacttgaaga tgtaccaggt ggagttcaag accatgaatg    1380

caaagctgct gatggatcct aagaggcaga tctttctaga tcaaaacatg ctggcagtta    1440

ttgatgagct gatgcaggcc ctgaatttca acagtgagac tgtgccacaa aaatcctccc    1500

ttgaagaacc ggatttttat aaaactaaaa tcaagctctg catacttctt catgctttca    1560

gaattcgggc agtgactatt gatagagtga tgagctatct gaatgcttcc taaaaagcga    1620

ggtccctcca aaccgttgtc atttttataa aactttgaaa tgaggaaact ttgataggat    1680

gtggattaag aactagggag ggg                                            1703

<210>  4
<211>  536
<212>  PRT
<213>  Homo sapiens

<400>  4

Met Gly His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser Val Pro Gly Val Gly Val Pro Gly
                325                 330                 335

Val Gly Ala Arg Asn Leu Pro Val Ala Thr Pro Asp Pro Gly Met Phe
            340                 345                 350

Pro Cys Leu His His Ser Gln Asn Leu Leu Arg Ala Val Ser Asn Met
        355                 360                 365

Leu Gln Lys Ala Arg Gln Thr Leu Glu Phe Tyr Pro Cys Thr Ser Glu
    370                 375                 380

Glu Ile Asp His Glu Asp Ile Thr Lys Asp Lys Thr Ser Thr Val Glu
385                 390                 395                 400

Ala Cys Leu Pro Leu Glu Leu Thr Lys Asn Glu Ser Cys Leu Asn Ser
                405                 410                 415

Arg Glu Thr Ser Phe Ile Thr Asn Gly Ser Cys Leu Ala Ser Arg Lys
            420                 425                 430

Thr Ser Phe Met Met Ala Leu Cys Leu Ser Ser Ile Tyr Glu Asp Leu
        435                 440                 445

Lys Met Tyr Gln Val Glu Phe Lys Thr Met Asn Ala Lys Leu Leu Met
    450                 455                 460

Asp Pro Lys Arg Gln Ile Phe Leu Asp Gln Asn Met Leu Ala Val Ile
465                 470                 475                 480

Asp Glu Leu Met Gln Ala Leu Asn Phe Asn Ser Glu Thr Val Pro Gln
                485                 490                 495

Lys Ser Ser Leu Glu Glu Pro Asp Phe Tyr Lys Thr Lys Ile Lys Leu
            500                 505                 510

Cys Ile Leu Leu His Ala Phe Arg Ile Arg Ala Val Thr Ile Asp Arg
        515                 520                 525

Val Met Ser Tyr Leu Asn Ala Ser
    530                 535

<210>  5
<211>  152
<212>  DNA
<213>  Simian virus 40

<400>  5
tagatcataa tcagccatac cacatttgta gaggttttac ttgctttaaa aaacctccca      60

cacctccccc tgaacctgaa acataaaatg aatgcaattg ttgttgttaa cttgtttatt     120

gcagcttata atggttacaa ataaagcaat ag                                   152

<210>  6
<211>  255
<212>  DNA
<213>  Escherichia coli

<400>  6
ttgagatcct ttttttctgc gcgtaatctg ctgcttgcaa acaaaaaaac caccgctacc      60

agcggtggtt tgtttgccgg atcaagagct accaactctt tttccgaagg taactggctt     120

cagcagagcg cagataccaa atactgttct tctagtgtag ccgtagttag gccaccactt     180

caagaactct gtagcaccgc ctacatacct cgctctgcta atcctgttac cagtggctgc     240

tgccagtggc gataa                                                      255

<210>  7
<211>  17
<212>  DNA
<213>  LAC Operator

<400>  7
ttgtgagcgg ataacaa                                                     17

## Description

The invention relates to plasmid expression vector encoding human interleukin 12 (IL-12) and its use for human cancer gene therapy using electroporation or any other applications. The said plasmid carries the human IL-12 fusion gene under the transcriptional control of human promoter of the *CDKN1A* (p21) gene. The plasmid carries no antibiotic resistance genes.

### BACKGROUND TO THE INVENTION

Existing treatment of cancer can be highly effective, but in many cases, it may fail to eradicate tumors completely. In case of melanoma, 3-5% of melanoma patients will eventually develop local recurrence or in-transit melanoma metastases, 5-13% regional disease and 3-10% distant disease [1]. In patients with malignant melanoma development of metastatic/systemic disease represents a great clinical challenge. The progression to systemic disease is highly dependent on the patient immune system.

Immune response can be boosted by the administration of pro-inflammatory cytokines that cause activation and proliferation of lymphocyte populations. To combat a metastatic disease, the immune system must recognize tumor cells throughout the body, which can be achieved by induction of a systemic immune response following recognition of tumor antigens in the primary tumor. Immune system must be boosted to recognize tumor associated antigens in the primary tumor. Cytokines are secretory molecules that regulate the intensity and duration of the immune response. One of the cytokines directing the immune response toward cellular or Th1 type, which is especially important in antitumor immune response, is interleukin 12 (IL-12).

### Interleukin 12

Many cytokines have been intensively studied as potential anticancer agents. Among them, IL-12 was proven to have strong antitumor activities. IL-12 is a secretory pro-inflammatory cytokine with multiple functions, including the induction of interferon-γ, activation of T helper and NK cells [2, 3], and antiangiogenic activity [4-6]. Recombinant IL-12 was proven to have potent antitumor and antimetastatic effects against murine tumors [7], yet its clinical application was hindered by dose-limiting toxicity associated by its systemic administration [8, 9]. Systemic treatment was therefore cancelled to be replaced with IL-12 gene therapy, which has already reached the clinical phase (reviewed in [10]). In recent years, several gene therapy studies in oncology have been performed with plasmid DNA encoding IL-12 and electroporation as a delivery system into the tumors or muscles for treatment of subcutaneous and visceral tumors in dogs and one preliminary study in human patients with subcutaneous melanoma nodules (reviewed in [11]). The results of the phase I/II melanoma clinical trial [12] demonstrated the safety of this approach and also clinical response in the treated and distant non-treated metastases.

Based on described experience with IL-12 electrogene transfer in preclinical studies [13, 14], and human [15] and veterinary [16] clinical studies, this therapy prolongs survival of cancer patients. The main advantage of the IL-12 gene therapy is that, in addition to potent local antitumor effect [17-20] it can also offer the systemic protection against distant metastases by induction of an effective immune response against tumor antigens or inhibiting angiogenesis of metastases [9, 15, 21] therefore it is especially suitable for the treatment of metastases which are responsible for more than 90% of cancer-associated deaths [22]. IL-12 gene therapy is robust in comparison to many new cancer treatments, where the trend in the last decade is the personalisation of the therapy. This means, it is suitable for the treatment of different types of metastatic cancer, independently of the mutation, and that no costly diagnostic methods are needed prior to the therapy.

### Electroporation - gene electrotransfer

One of the most promising non-viral techniques for delivery of gene material is electroporation with plasmid DNA or gene electrotransfer (GET). The method is generally safe [24], efficient, and can produce good reproducibility and spatial precision [23]. Advantage of plasmid vectors is that they are highly stable, convenient and inexpensive to produce, and flexible to design [25]. A further advantage is that plasmid DNA can have an adjuvant effect on the immune system provided by the presence of immunostimulatory sequences within the plasmid DNA. Namely, bacterially derived DNA contains oligonucleotide sequences that act to initiate the innate immune response and also stimulate maturation and migration of dendritic cells to lymph nodes, where they can interact with T cells of the adaptive immunity. The use of electroporation for the transfer of different plasmids encoding IL-12 has been disclosed in US 8026223 B1: Treating malignant tumors with high field strength electroporation of plasmids encoding IL-12.

### p21 promoter

The most commonly used vector for transferring gens using GET is plasmid DNA. Conventional promoters used to drive the expression of the therapeutic genes in plasmid vectors are constitutive, unregulated and ubiquitous. Usually they are of exogenous i.e. viral origin, for example CMV promoter, which renders them susceptible to methylation, meaning that they don't direct persistent transgene expression. Therefore, general tendency in gene therapy is to replace virus derived promoters with endogenous promoters, due to the safety concerns and longer lasting expression they support [26, 27]. One of the endogenous promoters, that also exert specificity to tumor cells, is the promoter of the *CDKN1A* gene. *CDKN1A* encodes the cyclin-dependent kinase inhibitor 1A protein (more commonly known as p21 or also as WAF1 or Cip1) which is a crucial regulator of the cell cycle, mediating cell cycle G1 phase arrest in response to stress. It also plays a role in cell death, DNA repair, senescence, aging and induced pluripotent stem cells reprograming [28]. Promoter of *p21* gene can be activated through p53-dependent [29] and also p53-independent way by various extrinsic stress stimuli including DNA damaging agents like irradiation and chemotherapeutic drugs, hypoxia and other intrinsic and oncogene stresses [30, 31]. The results of our preclinical study [32] showed that p21 promoter has a similar effectiveness as the constitutive promoter, but is safer. Promoter p21 is a native promoter of a gene that is present in all human cells; therefore it is not exogenous to the human body, meaning it doesn't get inactivated by methylation as it is often the case with the virus-derived promoters. The main advantage of the p21 promoter is its low basal activity in normal cells and high in cancer cells and its inducibility by different treatment induced stresses, so it is ideal for IL-12 gene therapy clinical studies. The potential use of p21 promoter has been disclosed in US 2002/142442 of Vogelstein and Kinzler (2002). (US 20020142442A1: Tumor suppressor WAF1).

### No AB resistance

Conventional plasmids carry antibiotic resistance gene needed for production of plasmid DNA in bacteria. In recent years safety concerns have been raised regarding this antibiotic resistance gene. There is a risk of spread of antibiotic resistance traits to environmental microbes and commensal bacteria by horizontal gene transfers which could provide pathogenic bacteria with antibiotic resistance. Also, if antibiotic selection is used during the production, residual traces of antibiotic used in their production can be found in the final product. This could be a problem for patients with hypersensitivity reactions to antibiotics which are relatively common [33]. Antibiotic resistance genes are, beside the safety concerns, responsible also for other drawbacks in plasmid DNA performance. Antibiotic resistance genes are large prokaryotic genes that hamper transfection efficiency due to the increased size of plasmid DNA [34] and they carry prokaryotic unmethylated CpG sequences, which can induce the innate immune system and cause inflammatory reactions [35].

According to the safety recommendation of the European Medicines Agency EMA (Reflection paper on design modifications of gene therapy medicinal products during development) antibiotics should be avoided during production of plasmids used in clinical trials.

Based on described experience with IL-12 electrogene transfer in preclinical studies, and human and veterinary clinical studies, this therapy prolongs survival of cancer patients. For that reason, what is needed in the art is a safe expression plasmid encoding an immune gene with proven antitumor efficacy, like IL-12, under a safer endogenic promoter and principally without AB resistance with the aim to transfer the knowledge from the preclinical studies to the clinical application also in EU.

### SUMMARY OF THE INVENTION

The present invention provides an expression plasmid comprising a polynucleotide encoding an IL-12 polypeptide and operably linked thereto a CDKN1A promoter, wherein said expression plasmid does not comprise a selectable marker gene.

The term "Interleukin 12" (abbreviated "IL-12") is well known in the art. Interleukin 12 is an interleukin that is naturally produced by dendritic cells, macrophages, neutrophils, and human B-lymphoblastoid cells (NC-37) in response to antigenic stimulation. In a preferred embodiment of the present invention, the term "Interleukin 12" refers to human Interleukin 12. The amino acid sequence of the human IL-12 polypeptide is well in in the art. Plasmids containing the IL-12 polynucleotide are commercially available (e.g. the pORFmIL12 plasmid, which is a commercially available plasmid from Invivogen).

In a particular preferred embodiment of the present invention, the human IL-12 polypeptide is encoded by a polynucleotide comprising a sequence as shown in SEQ ID NO:3. In particular, it is envisaged that the human IL-12 polypeptide comprises an amino acid sequence as shown in SEQ ID NO:4.

The term "Interleukin-12" preferably also encompasses variants of the aforementioned IL-12 polypeptide, in particular of the aforementioned human Interleukin-12 polynucleotide or polypeptide. Such variants shall share the same essential biological and immunological properties. Variants referred to above may be allelic variants or any other species specific homologs, paralogs, or orthologs. In particular, a variant shall preferably have at least 60%, 70%, 80%, 85%, 90%, 95%, 97%, 98%, or 99% identity with the amino sequence of the specific IL-12 polypeptide, preferably over the entire length of said polypeptide. The degree of identity between two amino acid sequences can be determined by algorithms well known in the art. Preferably, the degree of identity is to be determined by comparing two optimally aligned sequences over a comparison window. Optimal alignment of sequences for comparison may be conducted by the homology alignment algorithm of Needleman and Wunsch [36], or by computerized implementations of alignment algorithms (GAP, BESTFIT, BLAST, PASTA, and TFASTA in the Wisconsin Genetics Software Package, Genetics Computer Group (GCG), 575 Science Dr., Madison, Wis.), or by visual inspection. Preferably, the default values of 5.00 for gap weight and 0.30 for gap weight length are used.

The polynucleotide encoding the IL-12 polypeptide shall be operably linked to a CDKN1A promoter. The term "operably linked" is well understood by the skilled person. As used herein the term refers to the linkage of the CDKN1A promoter upstream from the polynucleotide encoding the IL-12 polypeptide such that the promoter mediates transcription of the polynucleotide encoding said IL-12 polypeptide. Thus, the expression plasmid of the present invention shall comprise an expression cassette comprising a polynucleotide encoding the IL-12 polypeptide operably linked to the CDKN1A promoter.

The CDKN1A promoter is the promoter of the CDKN1A gene. CDKN1A encodes the cyclin-dependent kinase inhibitor 1A protein (frequently also refer to as p21 or also as WAF1 or Cip1) which is a crucial regulator of the cell cycle, mediating cell cycle G1 phase arrest in response to stress. The potential use of the CDKN1A promoter (or p21 promoter) has been disclosed in US 2002/142442 which herewith is incorporated by reference in its entire disclosure content.

In a preferred embodiment, the CDKN1A promoter is the human CDKN1A promoter. The sequence of this promoter is well known in the art. In a particular preferred embodiment, the human CDKN1A promoter comprises a sequence as shown SEQ ID NO:2.

The term "CDKN1A promoter" also encompasses variants of the aforementioned promoter, in particular of the aforementioned human CDKN1A promoter. Such variants shall have essentially the same activity as the human CDKN1A promoter. In particular, a variant shall have at least 60%, 70%, 80%, 85%, 90%, 95%, 97%, 98%, or 99% identity with the nucleic acid sequence of the specific CDKN1A promoter, preferably over the entire length of said promoter (in particular of the promoter used in the Examples section). The term "variant" also encompasses fragments of the aforementioned human CDKN1A promoter which retain the promoter activity of the aforementioned human CDKN1A promoter.

Preferably, the used promoter is un-optimized. An un-optimized promoter is preferably a promoter having a native sequence, in particular sequence which naturally occurs in humans.

In an embodiment of the expression plasmid of the invention, the expression cassette comprised by the plasmid further comprises a terminator sequence. The term "terminator" is well known in the art and refers to a nucleic acid sequence at the end of a transcriptional unit which signals termination of transcription. Preferably, the terminator allows for terminating transcription in mammalian cells, in particular human cells. In an embodiment, the terminator is of mammalian, in particular human origin. In another embodiment, the terminator is of viral origin. For example, the terminator may be the SV40 terminator. The sequence of the SV40 terminator is shown in SEQ ID NO:5.

The term "plasmid" as used herein preferably refers to a linear or, in particular, to a circular DNA vector that is capable of transforming bacteria and that is capable of replicating episomally in the transformed bacteria, in particular in *E.coli* cells. In a preferred embodiment of the expression plasmid of the present invention, the plasmid comprises an origin of replication. The origin of replication shall allow for replication in bacterial cells, in particular in E. coli cells such as the pUC origin of replication. Preferably, the expression plasmid is present in a high copy number in bacterial cells. Thus, it is envisaged that the plasmid is a high copy plasmid. This can be achieved by using an origin of replication which allows for a high copy number such as the pUC origin of replication. The sequence of a preferred origin of replication is shown in SEQ ID NO:6.

As set forth above, expression plasmid of the present invention shall not comprise a selectable marker gene. As used herein, the term "selectable marker gene" preferably refers to a polynucleotide encoding a polypeptide which preferably confers resistance to a selection agent such as an antibiotic. In a preferred embodiment, the expression plasmid does not comprise a bacterial selectable marker gene. In a particular preferred embodiment, the expression plasmid which does not comprise a selectable marker gene does not comprise an antibiotic resistance gene.

In accordance with the present invention, it is contemplated that the expression plasmid does not comprise a polynucleotide encoding for a polypeptide except for the polynucleotide encoding for the IL-12 polypeptide. In particular, the plasmid shall not comprise a polynucleotide which encodes for a polypeptide having a length of more than 100 amino acids or 150 amino acids (except for the polynucleotide encoding for the IL-12 polypeptide).

An expression plasmid of the present invention that does not comprise a selectable marker gene can be prepared using ORT technology (Cobra Biologics Ltd). The ORT technology is well known in the art and e.g. described in WO 1997009435 A1 which herewith is incorporated by reference with respect to its entire disclosure content. The technology employs a plasmid-mediated repressor titration to activate a host selectable marker, removing the requirement for a plasmid-borne marker gene. Current ORT® strains are engineered to contain an essential gene, dapD under transcriptional control of the lac operator/promoter (IacO/P), although any essential gene could be used. In the absence of an inducer such as IPTG, this strain cannot grow due to the repression of dapD by the Lacl repressor protein binding to IacO/P. Transformation with a high copy number plasmid containing the lac operator (IacO) effectively induces dapD expression by titrating Lacl from the operator. Regulation of the essential gene ensures the growth of bacteria and maintenance of recombinant plasmids containing IacO and an origin of replication. ORT® is used along with X-mark™, which enables a plasmid to be constructed using highly efficient antibiotic selection in a special E. coli strain, then transformed into an ORT® strain, which rapidly excises the antibiotic resistance gene by Xer recombination to generate a 1kb smaller plasmid, which is stably maintained by ORT®. This greatly accelerates the time for generating plasmids in ORT® strains (ORT). ORT technology was used before for the preparation of plasmids used for the DNA vaccination (Reviewed in Cranenburgh, 2013) and for the preparation of plasmid antiangiogenic metargidin peptide (AMEP) (US 20100197769 A1: Plasmid containing a sequence encoding a disintegrin domain of metargidin (rdd)) that was also used in a clinical trial in combination with electrogene transfer (Spanggaard, 2013). In addition to plasmid DNA production, the ORT® system has been adapted for vaccine delivery using live bacterial vectors as ORT-VAC by Prokarium Ltd.

Thus, the presence of the lac operator sequence in the expression plasmid of the present invention allows for enriching the expression plasmid in a suitable host cell (such as an *E.coli* cell comprising the dapD gene under transcriptional control of the lac operator/promoter). In an embodiment, the expression plasmid of the present invention thus further comprises a lac operator sequence.

The term "Lac operator sequence" refers to a DNA sequence that is bound by the lac repressor. Sequences of lac operators are well known in the art. In an embodiment of the present invention, the lac operator sequence comprises a sequence as shown in SEQ ID NO:7.

In a preferred embodiment, the expression plasmid comprises a sequence as shown in SEQ ID NO:1. The sequence of this plasmid is also shown in Figure 8.

The present invention further relates to host cell comprising the expression plasmid of any one of claims 1 to 6. In a preferred embodiment, the host cell further comprises an essential gene operably linked the lac operator/promoter (IacO/P). Said essential gene can be any gene which essential for the growth of the cell. Preferably, said gene is the *dapD* gene which is a gene encoding for a 2,3,4,5-tetrahydropyridine-2,6-dicarboxylate N-succinyltransferase (EC 2.3.1.117). In an embodiment, said gene is a gene which is heterologous with respect the cell.

Preferably, the host cell is an E. coli cell.

The present invention further relates to a method for producing the expression plasmid of any one of claims 1 to 6, said method comprising:
a) cultivating the host cell as set forth above, and
b) extracting the expression plasmid from said host cell.

In the studies underlying the present invention, the plasmid encoding the human IL-12 fusion gene covalently linked to p21 (CDKN1A) promoter was prepared with standard molecular cloning methods and confirmed by restriction analysis. The human IL-12 fusion gene originates from the commercially available pORF-hIL-12 G2 plasmid (InvivoGen, pORF-hIL-12 G2) and the coding sequence for p21 originates from WWP-Luc plasmid from Bert Vogelstein's lab (now available at Addgene). The expression cassette with therapeutic gene under the control of inducible promoter was sub-cloned in a vector without any selection marker using the antibiotic-free maintenance system ORT® and X-mark™ (Cobra Biologics Pic). The constructed plasmid was evaluated for stability and its nucleotide sequence was confirmed by restriction analysis and sequencing. The functionality of the plasmid was evaluated in *in vitro* and *in vivo* experiment which is described in more details in the Examples given under the Description of an embodiment. The expression profile of the plasmid was determined by quantification of IL-12 expression at mRNA level using a quantitative reverse transcription polymerase chain reaction (qRT-PCR) and protein level using ELISA assay. Additionally, the toxicity of the plasmid was determined after the gene electrotransfer of p21-hIL-12ORT in B16F10 mouse melanoma model. Blood chemistry and hematology changes, histology changes and antitumor response were evaluated.

The said plasmid can be readily produced by one skilled in the art. Milligram quantities of plasmid DNA can be produced by inoculating an appropriate culture medium, such as LB medium, with the transformed E. coli strain and culturing in conventional laboratory flasks at 37°C with shaking (for instance, at 200 rpm). Small quantities of the overnight culture can be used to prepare glycerol-amended frozen stocks of the transformed plasmid-carrying bacteria that can be stored at -80°C for years, for use for subsequent inoculations of the LB media. The plasmid can also be produced in industrial-scale fermenters. The plasmid can be recovered from the overnight bacterial culture using an appropriate method for purification of the plasmid, for instance, plasmid isolation kits or any other method that are well known in the art. After the plasmid is isolated, its identity, quality, and quantity can be determined using any method well known in the art, such as spectrophotometry, restriction analysis, and sequencing. A method for production and purification of the plasmid, and confirmation of its identity, is further described in Example 1.

There are two advantages of the plasmid over previously used plasmids (described in the background): firstly, no AB resistance, which is allowed for use in clinical studies by the EMA, and secondly, virus CMV promoter is replaced with endogenous native un-optimized p21 promoter. In already mentioned concluded human clinical trial a plasmid containing a human IL-12 gene under the control of a human cytomegalovirus (CMV) promoter, containing also AB resistance gene, was employed (12). In our plasmid the exogenous (virus) constitutive promoter (CMV) was replaced with the endogenous (human) promoter of the *CDKN1A* gene also known as *p21.* The advantages of this promotor include: 1) induction of the expression of the downstream IL-12 gene by genotoxic stress- like the one induced during chemotherapy or radiotherapy treatment; 1) induction with tumors specific conditions, for instance hypoxia; 3) since the promoter is endogenous to the eukaryotic cells, its transcriptional inactivation should be lower, compared to the viral promoters; 4) since the p21 promotor used is un-optimized, it also contains immunostimulative sequences that can have an adjuvant effect on the therapy efficacy.

As set forth above, the expression plasmid of the present invention can be used for the treatment of cancer.

Accordingly, the present invention relates to a pharmaceutical composition comprising the expression plasmid of the present invention. Preferably, the pharmaceutical composition comprises the expression plasmid in a therapeutically effective amount.

In addition, the present invention relates to the expression plasmid of the present invention for use in the treatment of cancer.

Furthermore, the present invention relates to the use of the expression plasmid of the present invention for the manufacture of a medicament for the treatment of cancer.

In addition, the present invention relates to method for the treatment of cancer, comprising administering a therapeutically effective amount of the expression plasmid of the present invention to a patient suffering from cancer.

According to the present invention, a patient shall be treated with the expression plasmid. Said patient is preferably an animal, more preferably a mammal such as a primate or a rodent such as a mouse or a rat, and most preferably a human. It is to be understood that the patient to be treated in accordance with the present invention shall suffer from cancer.

The terms "treatment of cancer" or "treatment of a tumor", preferably, refer to therapeutic measures, wherein the object is to prevent or to slow down an undesired physiological change or disorder. It is to be understood that a treatment can also mean prolonging survival as compared to expected survival if not receiving treatment.

The term "cancer" is well known in the art. In particular, the term refers to a proliferative disorder or disease caused or characterized by the proliferation of cells which have lost susceptibility to normal growth control. Preferably, the term encompasses tumors and any other proliferative disorders. In particular, the term "cancer" refers to a tumor. Accordingly, the present invention contemplates the treatment of a tumor.

In a preferred embodiment, the cancer is sarcoma, carcinoma, lymphoma, melanoma or haemangioma. In a particular preferred embodiment, the cancer to be treated is melanoma.

The treatment as set forth herein shall be gene therapy. Thus, exogenous genetic sequences, i.e. the expression plasmid of the present invention shall be introduced into patients with the aim of the treatment of cancer. In an embodiment, the gene therapy is carried out *in vivo,* i.e. the plasmid or pharmaceutical composition of the present invention is administered directly to the patient. In an alternative embodiment, the plasmid or pharmaceutical composition is administered *ex vivo.*

In connection with the present invention, the expression plasmid or pharmaceutical composition of the present invention is preferably administered peritumorally or, in particular intratumorally.

The term "intratumoral administration" means that the expression plasmid or pharmaceutical composition is directely administered into a tumor. The term "peritumoral administration" means that the expression plasmid or pharmaceutical composition is administered into tissue surrounding a tumor. This is e.g. carried out after surgical removal of a tumor.

In a preferred embodiment, the administration, in particular the intra- or peritumoral administration, is carried out by injection, such as by injection with a needle and a syringe. For injection, the expression plasmid is preferably present in solution, such as in water, phosphate buffered saline, citrate buffer, or Tris-HCl buffer. Thus, the pharmaceutical composition of the present invention may be provided in form of such a solultion.

The uptake of the expression plasmid or pharmaceutical composition of the present invention into cells of the patient to be treated may be supported by means know in the art. In the most preferred embodiment of the invention, the said plasmid is transfected in the host cells (either in vitro or in vivo) by electroporation. Any other standard method for introduction of plasmid DNA in the host cells, may also be used, e. g. lipofection, magnetofection, sonoporation, microinjection, etc.

Accordingly, the administration, and thus the uptake into the cells of the patient, is achieved preferably by electroporation. Preferably, the plasmid is transfected into cells of the patient, in particular cells comprised by the tumor or cells that are located near to the tumor, by electroporation.

The electroporation shall follow the administration. Thus, it is envisaged to administer the expression plasmid or pharmaceutical composition of the present invention to the patient (as described above), followed by electroporation. For example, the expression plasmid or pharmaceutical composition is injected as described above. Subsequently, e.g. 0 to 10 minutes after administration, the plasmid is transfected by electroporation.

Gene electroporation is frequently also referred to as gene electrotransfer (GET). How to transfect cells by electroporation is well known in the art. E.g., the use of electroporation for the transfer of different plasmids encoding IL-12 has been disclosed in US 8026223 B1 which herewith is incorporated by reference in its entirety. The method is generally safe (Spanggaard 2012), efficient, and can produce good reproducibility and spatial precision. In addition, electroporation is described in the Examples section. For transfection of plasmid DNA via electroporation, tumors are usually placed between two parallel stainless steel electrodes with e.g. a 6 mm gap and EP is applied (e.g. 8 square-wave EP of amplitude 600 V/cm, duration 5 ms and frequency 1 Hz given in two perpendicular directions). The EP can be generated by electric pulse generator ELECTRO CELL B10 (Betatech).

The expresssion plasmid of the present invention can be administered once or more than once. Thus, it can be repeated several times to achieve effective dose and antitumor effectiveness.

Preferably, between 5 to 4000 µg of the expression plasmid are administered, more preferably 500 to 3000 µg, and more preferably 1000 to 2000 µg. This applies in particular to a single dose.

The plasmid is suitable for use in human gene therapy. Since it encodes an immune molecule it is predominantly meant to be used as part of an adjuvant immune gene therapy in combination with standard treatments in oncology, such as surgical tumor excision or debulking, radiotherapy, systemic treatments and electrochemotherapy, by adding a systemic immune-mediated antitumor effect to these therapies.

Thus, the treatment with the plasmid of the present invention may be carried out in addition to a further cancer therapy. Preferably, said further cancer therapy is selected from radiotherapy, chemotherapy, including electrochemotherapy, targeted therapy and immunotherapy. Again, it is envisaged that expression plasmid or pharmaceutical composition of the present invention can be administered intratumorally or, in particular, peritumorally.

Aspects and embodiments of the present invention will now be illustrated, by way of example, with reference to the accompanying figures. Further aspects and embodiments will be apparent to those skilled in the art. All documents mentioned in this text are incorporated herein by reference.

### BRIEF DESCRIPTION OF THE FIGURES AND TABLES

Embodiments and experiments illustrating the principles of the invention will now be discussed with reference to the accompanying figures.

The figures show:

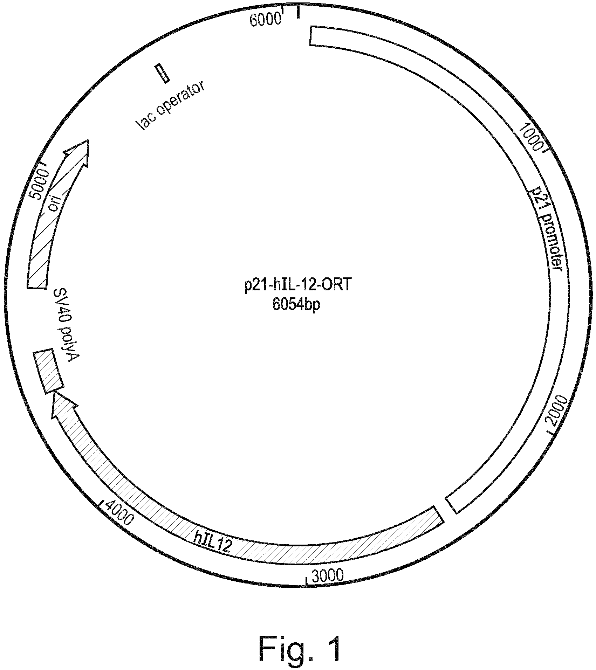

- **Fig. 1**: Map of the plasmid encoding the human IL-12 under transcriptional control of the p21 promoter and without the gene for antibiotic resistance;

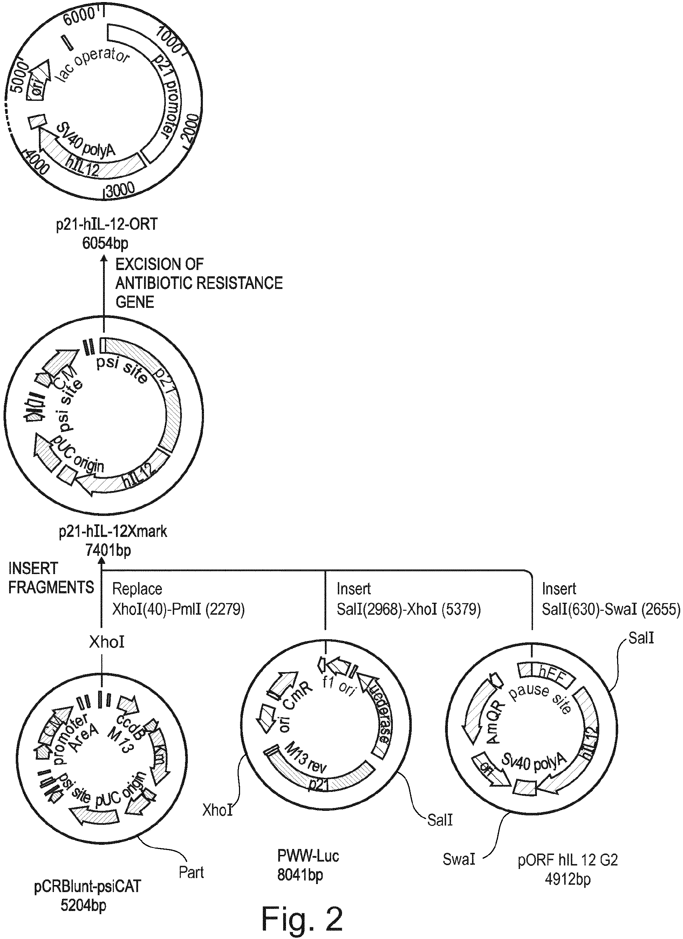

- **Fig. 2**: A schematic representation of the construction of the plasmid encoding human IL-12 under transcriptional control of the p21 promoter and without the gene for antibiotic resistance;

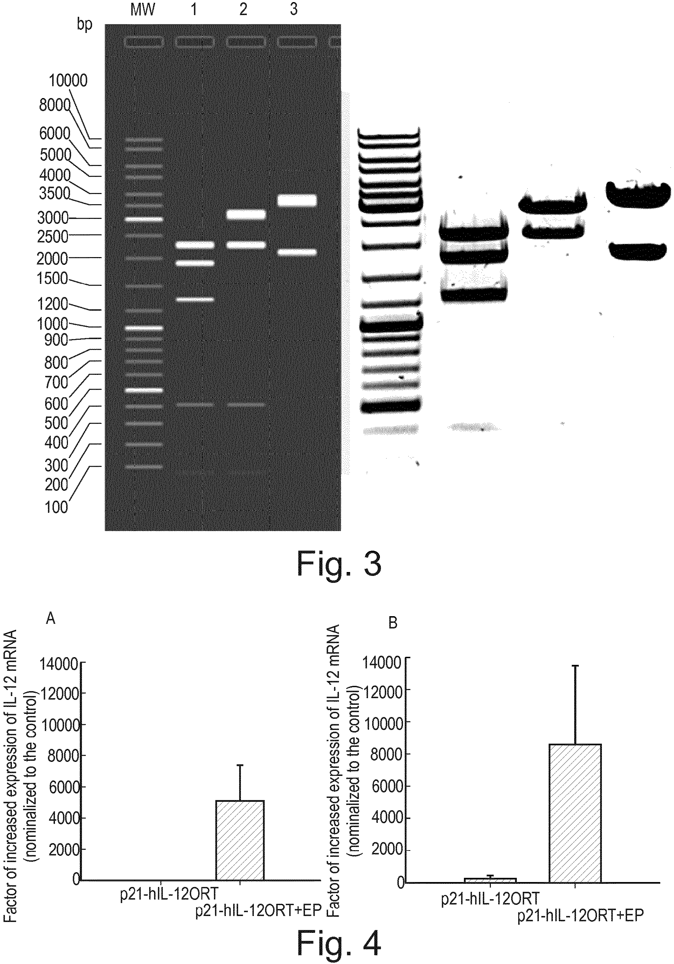

- **Fig. 3**: Restriction analysis confirming the identity, purity, and concentration of the plasmid (MW: GeneRuler DNA^{tm} DNA Ladder Mix, Lane 1: HindIII + Mun = 2338 bp, 1925 bp, 1338 bp, 409 bp, 79 bp, 19 bp, Lane 2: Hindlll = 3260 bp, 2338 bp, 409 bp, 79 bp, 19 bp, Lane 3: Kpn = 3966 bp, 2142 bp;
- **Fig. 4**: Expression of the human IL-12 on mRNA level after gene electrotransfer of the p21-hIL-12ORT. (A) B16F10 melanoma cell and intratumoral (B) hIL-12 mRNA levels 1 day after GET of p21-hIL-12ORT. *In vitro* experiments were repeated three times independently. *In vivo*, 6 animals per experimental group;

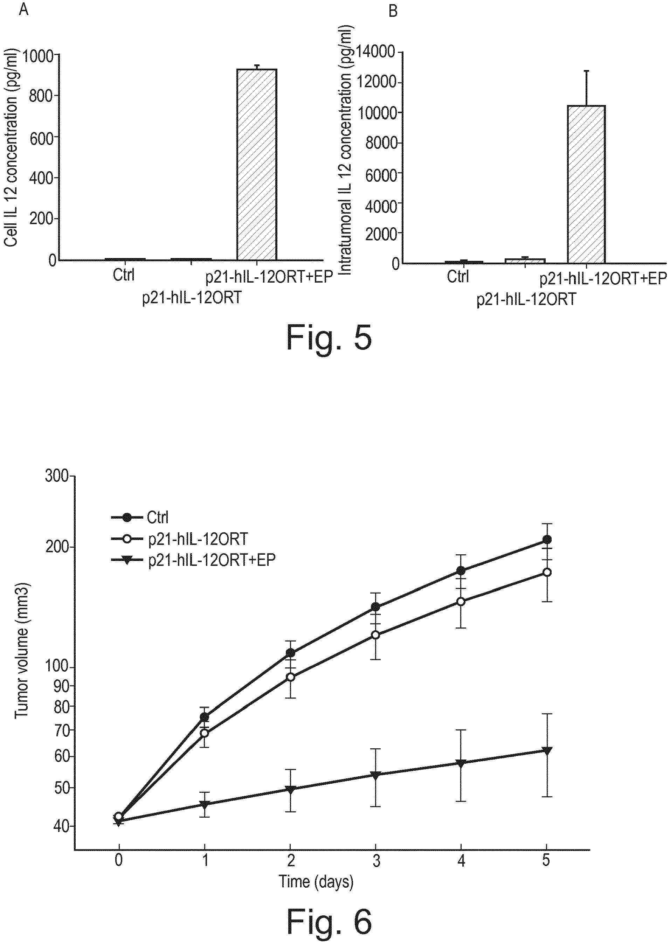

- **Fig. 5**: Expression of the human IL-12 on protein level after gene electrotransfer of the p21-hIL-12ORT. (A) B16F10 melanoma cell media and intratumoral (B) concentration of hIL-12 protein 3 days after GET of p21-hIL-12ORT. *In vitro* experiments were repeated three times independently. *In vivo*, 6 animals per experimental group. *, P < 0.05 vs. Ctrl and p21-hIL-12ORT;
- **Fig. 6**: Antitumor effect of gene electrotransfer of p21-hIL-12ORT in mouse B16F10 melanoma. Growth of B16F10 tumors exposed to a single therapy: intratumoral injection of H₂O alone (Ctrl), plasmid alone (p21-hIL-12ORT) or in combination with application of electric pulses (p21-hIL-12ORT +EP); and

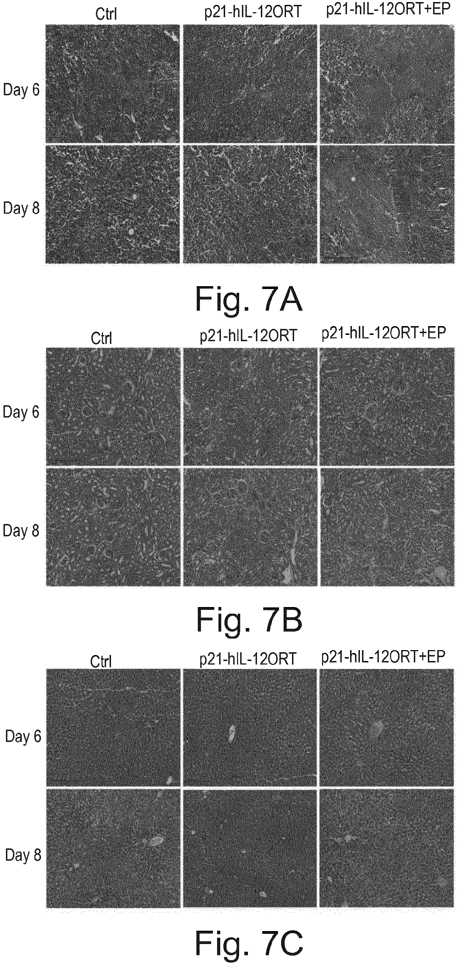

- **Fig. 7**: Gene electrotransfer of p21-hIL-12ORT increase necrosis of mouse B16F10 melanoma tumors, with no effect on tissue changes in kidney and lung. Tumor necrosis (A) and histology changes in kidney (B) and lungs tissue (C) after gene electrotransfer of p21-hIL-12 in mouse B16F10 melanoma tumors *in vivo.* Representative images taken at days 6 and 8 with a DP72 CCD camera (Olympus) connected to a BX-51 microscope (Olympus). Scale bar 200 µm.

### DESCRIPTION OF AN EMBODIMENT:

Preclinical evaluation of the toxicity following the delivery of the plasmid by EP in B16F10 mouse melanoma model.

In the following description of an embodiment, reference is made to the accompanying drawings, which form a part hereof, and within which are shown by way of illustration specific embodiments by which the invention may be practiced. It is to be understood that other embodiments may be utilized and structural changes may be made without departing from the scope of the invention.

### Examples

### Example 1 - Production and purification of the plasmid

Transformed bacterial cells were cultured in LB culture medium overnight at 37°C at 300 rpm. The next day 1 mL of bacterial culture was used to prepare glycerol stocks by adding glycerol to the final concentration of 25% and snap freezing in liquid nitrogen. The rest of the bacterial culture was used to recover the plasmid using the megaprep endotoxin free plasmid isolation kit (EndoFree Plasmid Mega Kit, Qiagen Germany)). The isolated plasmid was then subjected to the spectrophotometric determination of concentration and purity (Epoch Microplate Spectrophotometer, Bio-Tec) and restriction analysis. The plasmid was cut different combinations of restriction enzymes (Thermo Fisher Scientific, Thermo scientific, USA) with restriction sites at different parts of the plasmid and the identity of the plasmid was confirmed by positive matching of the pattern of bands on the electrophoresis gel to the expected pattern obtained by a simulation experiment.

### Construction of the therapeutic plasmid with p21 promoter without the gene for antibiotic resistance: results

Recombinant plasmid encoding hIL-12 under the transcriptional control of p21 promoter and without the gene for antibiotic resistance was successfully constructed (**Fig. 1**) using standard molecular biology techniques of restriction and ligation (**Fig. 2**). Construction of p21-hIL-12ORT was confirmed by restriction analysis on agarose gel electrophoresis (**Fig. 3**) and by complete plasmid sequencing (CCIB DNA core, Massachusetts general hospital, USA) (SEQ ID NO:1).

### Example 2 - Validation of the plasmid expression profile in vitro and in vivo.

The expression profile of the plasmid was determined by quantification of IL-12 expression at mRNA level using a quantitative reverse transcription polymerase chain reaction (qRT-PCR) and protein level using ELISA assay.

Murine B16F10 melanoma cells were trypsinized and washed in DMEM with 5% FBS. After 5 minutes centrifugation, the cell pellet was washed in ice-cold electroporation buffer (125 mM sucrose, 10 mM K2HPO₄, 2.5 mM KH₂PO₄, 2 mM MgCL₂) and resuspended at a concentration of 2.5 x 10⁷ cells/ml. The cells (1 x 10⁶) were then mixed with 10 µg of p21-hIL-12ORT and placed between two parallel plate stainless steel electrodes with a 2 mm gap, connected to a homemade electroporator (Faculty of Electric Engineering, University of Ljubljana, Ljubljana, Slovenia) and subjected to 8 square-wave electric pulses (EP) at 100 V for a duration of 5 ms and at a frequency of 1 Hz. Immediately after GET, 50 µl of 100% FBS was added to the cells, which were incubated for 5 min at room temperature and then plated onto 10 cm² Petri dishes (Greiner Bio-One, Germany) for further assays.

Female C57BL/6 mice, 6-8 weeks old were obtained from Harlan Laboratories (Harlan Laboratories, Udine, Italy). The mice were maintained in a specific pathogen-free colony at constant room temperature (21°C) and a 12 hours light/dark cycle. Water and food were provided *ad libitum.* All experiments were performed in accordance with the guidelines of the EU directive and the permission from the Republican Ethics Committee, which operates under the auspices of the Veterinary Administration of the Ministry of Agriculture, Forestry and Food of the Republic of Slovenia (permission no.: 34401-1/2015/7).

B16F10 tumors were induced by subcutaneous inoculation in the right flank of the mice of 1 x 10⁶ viable tumor cells in 0.1 ml of 0.9% sodium chloride (NaCl), which were prepared from cell culture *in vitro.* When tumors reached volume of 40 mm³, they were treated with intratumoral injection of 50 µg/50µl of plasmid p21-hIL-12ORT. After 10 minutes, tumors were placed between two parallel stainless steel electrodes with a 6 mm gap and EP were applied (8 square-wave EP of amplitude 600 V/cm, duration 5 ms and frequency 1 Hz given in two perpendicular directions). The EP were generated by electric pulse generator ELECTRO CELL B10 (LEROY biotech, France). The following groups were included in the experiment: Injection of H₂O alone (Ctrl), intratumoral injection of p21-hIL-12ORT alone (p21-hIL-12ORT) or in combination with EP (p21-hIL-12ORT+EP). In each group, 5-6 mice were included.

For *in vitro* and *in vivo* determination of IL-12 mRNA levels, total RNA was isolated from the cells and tumors 1 day after gene electrotransfer of p21-hIL-12ORT using the TRIzol Plus RNA Purification System (Thermo Fisher Scientific , Invitrogen, USA). One µg of total RNA was reverse transcribed into cDNA using SuperScript VILO^{™} cDNA Synthesis Kit (Thermo Fisher Scientific, Invitrogen, USA), according to the manufacturer's instructions. Two µl of the 10-times and 100-times diluted mixture was used as the template for the qRT-PCR using SYBR® Green PCR Master Mix (Thermo Fisher Scientific, Applied Biosystems, USA). A pair of primers was used to amplify hIL-12 and the housekeeping gene *glyceraldehyde 3-phosphate dehydrogenase* (GAPDH). qRT-PCR was performed on 7300 System (Thermo Fisher Scientific , Applied Biosystems, USA) as follows: activation of AmpliTaq Gold Enzyme (10 min at 95°C), 45 cycles of denaturation (15 s at 95°C), annealing and extension (1 min 60°C). The level of hIL-12 expression in each sample was calculated as ratio of hIL-12 vs. reference gene GAPDH mRNA and normalized to untreated control group.

To determine the protein concentration of IL-12, Quantikine® ELISA Human IL-12 p70 Immunoassay (R&D Systems, USA) was performed according to the manufacturer's instructions. For *in vitro* quantification, whole media from B16F10 melanoma cells was removed 3 days after gene electrotransfer of p21-hIL-12ORT, centrifugated and aliquots were stored at -80°C for further processing. For *in vivo* quantification, mice were sacrificed and tumors were removed, weighed and stored at -80°C. Immediately after mechanical maceration of tumors in liquid nitrogen, tumor samples were resuspended with 500 µl of PBS containing protease inhibitors and centrifuge for 10 min at 10,000 rpm. The supernatant was separated from the sediment and stored at -80°C until analysis. Concentrations of IL-12 were determined from the slope of standard curve and calculated as pg of cytokine per mL of media or ng of cytokine per mg of tumor tissue (**Fig. 4**).

### Validation of the plasmid expression profile in vitro and in vivo: results

In order to determine the expression of hIL-12 on mRNA and protein level, B16F10 melanoma cells, as well as tumors, were transfected with p21-hIL-12ORT using GET. Expression of hIL-12 on mRNA level was determined by qRT-PCR 1 day after GET and the concentration of hIL-12 protein was measured 3 days after GET using ELISA assay.

The expression of hIL-12 on mRNA level in B16F10 melanoma cells and tumors was statistically significantly higher after GET of the p21-hIL-12ORT (**Fig. 4**). Furthermore, intratumoral and cell media concentration of hIL-12 protein were also statistically significantly higher in B16F10 melanoma cells and tumors after GET of the p21-hIL-12ORT (**Fig. 5**).

### Example 3 - Toxicity of the plasmid

The toxicity of the plasmid was evaluated after the gene electrotransfer of p21-hIL-12ORT in B16F10 mouse melanoma model. Blood chemistry and hematology changes, histology changes and antitumor response were evaluated.

For determination of hematology and chemistry changes, blood samples were collected from infraorbital sinus from individual mice from each experimental group 1, 3, 6 and 8 days post-treatment. Blood was stored at room temperature for approximately 2 h and then hematology analysis was performed (**Table 1**). For determination of chemistry changes, blood samples were stored for a 20 min at 4°C; serum was extracted by centrifugation at 3,000 rpm for 5 min and stored at -20°C until analysis (**Table 2**). Blood and serum were analyzed and compared with the normal values.

The antitumor response of gene electrotransfer of p21-hIL-12ORT was determined using the tumor growth delay assay. Tumors were measured in three perpendicular directions (a, b, c) using a digital caliper. Tumor volume was calculated by the formula: V = a × b × c × π/6 (**Fig. 6**). Tumor growth was followed until the tumors reached an average volume of 350 mm³, after which the mice were sacrificed.

For histological analysis, mice were sacrificed 6 and 8 days post-treatment and their kidney, lungs and tumors were excised. Tumors were fixed in IHC zinc fixative (BD Pharmingen, BD Biosciences) for 24 hours and then embedded in paraffin. Tissue sections of 2 µm were cut from each paraffin block and used for hematoxylin and eosin (HE) staining. Images from each tissue slide were obtained under visible light at 20 x objective magnification, with a DP72 CCD camera (Olympus) connected to a BX-51 microscope (Olympus) (**Fig. 7**).

### Toxicity of the p21-hIL-12ORT: results

To determine the toxicity of the p21-hIL-12ORT, B16F10 melanoma tumors were transfected with p21-hIL-12ORT using GET. Tumor growth was followed; blood was collected 1, 3, 6 and 8 days post- treatment in order to determine blood chemistry and hematology changes. To evaluate histology changes, kidneys, lungs and tumors were excited 6 and 8 days post-treatment.

After a single GET of p21-hIL-12ORT in B16F10 melanoma tumors *in vivo,* reduction of tumor growth compared to all other groups was observed in first 5 days post-treatment (**Fig. 6**). Tumors from experimental group that received p21-hIL-12ORT only grew at the same growth rate as tumors from the untreated control group.

Differences in some standard hematology categories were observed in all experimental groups regardless of the time point blood collection post-treatment (**Table 1**). In mice from all experimental groups, erythrocytes (RBC) were slightly increased, while hemoglobin (HGB) was increased only in untreated mice at day 1 post-treatment. However, statistically significant decrease of RBC and HGB was observed in untreated mice and mice from pertinent control group at day 8 post-treatment. Hematocrit (HCT) was increased in mice from all experimental groups at days 1 and 3 post-treatment, while a statistically significant HCT decrease was observed in untreated mice at day 8. Changes in a slight increased mean corpuscular volume (MCV) were observed in mice from pertinent control group and treated group at later time point.

Mice from all experimental groups showed decreased mean corpuscular hemoglobin concentration (MCHC), mean cell hemoglobin concentration (CHCM) and cell hemoglobin (CH) at all time points post-treatment with no statistically significant differences between the groups. Red cell distribution (RDW) and hemoglobin distribution width (HDW) were increased but only at later time point.

Blood chemistry analysis showed that creatinine and total serum protein values were not beyond the limit of normal values after GET of p21-hIL-12ORT (**Table 2**). Additionally, these values were also almost unchanged in mice from untreated and pertinent control group, receiving plasmid injection only. Albumin values in serum samples, taken from mice from all experimental groups at day 1, 6 and 8 post-treatment, were slightly higher compared to the normal values. However, there were no statistically significant changes in albumin values between the experimental groups.

### Tables

The following tables show:
- **Table 1:**: **Blood hematology changes 1, 3, 6 and 8 days post-treatment after gene *electrotransfer* of p21-hIL-12 to B16F10 mouse melanoma tumors *in vivo. Intratumoral injection of H₂O alone (Ctrl), injection of* plasmid *alone (p21-hIL-12) or in combination with application of electric pulses (p21-hIL-12+EP).***
- **Table 2:**: **Blood chemistry changes 1, 6 and 8 days post-treatment after gene *electrotransfer* of p21-hIL-12ORT to B16F10 mouse melanoma tumors *in vivo. Intratumoral injection of H₂O alone (Ctrl), injection of* plasmid *alone (p21-hIL-12) or in combination with application of electric pulses (p21-hIL-12+EP).***

In **Table 1** following abbreviations are used: WBC, White blood cell; RBC, Red blood cell; HGB, Hemoglobin; HTC, Hematocrit; MCV, Mean corpuscular volume (reflect average volume of red cells); MCH, Mean corpuscular hemoglobin; MCHC, Mean corpuscular hemoglobin concentration; CHCM, Cell hemoglobin concentration mean; CH, cell hemoglobin; RDW, Red cell distribution width; HDW, Hemoglobin distribution width; PLT, Platelet count; MPV, Mean platelet volume; NEUT, Neuthrophils; LYMPH, Lymphocytes; MONO, Monocytes; eosinophils; BASO, Basophils; LUC, Leukocytes; AM ± SE, arithmetic means ± standard error of the mean. **P* < 0.05 compared to all groups at day 1; ***P <* 0.05 compared to Ctrl, p21-hIL-12ORT+EP at day 1 and Ctrl at day 3; +*P* < 0.05 compared to Ctrl, p21-hIL-12ORT+EP at day 1; ++*P<* 0.05 compared to all groups at day 1, Ctrl at day 3 and p21-hIL-12ORT at day 6;
wherein underlined highlighting indicates values outside reference values.

In **Table 2** following abbreviations are used: TP2, Total serum protein; AM ± SE, arithmetic means ± standard error of the mean;
wherein underlined highlighting indicates values outside reference values.

| **Table 1** | | | **Day 1** (AM±SE) | | |
|---|---|---|---|---|---|
| TEST | NORMALS | UNITS | Ctrl | p21-hIL-12ORT | p21-hIL-12ORT+EP |
| WBC | 3.2-12.7 | X10⁹/L | 4.7±1.1 | 5.3±0.7 | 3.7±0.7 |
| RBC | 7.0-10.1 | X10¹²/L | 10.5±0.1 | 10.4±0.3 | 10.4±0.1 |
| HGB | 118-149 | g/L | 155.2±1.6 | 125.5±28.4 | 153.7±2.3 |
| HCT | 36.7-46.8 | L/L | 57.0±1.3 | 55.8±2.1 | 56.5±0.9 |
| MCV | 42.2-59.2 | fL | 54,1±0.8 | 53.2±0.6 | 54.5±0.4 |
| MCH | 13.8-18.4 | pg | 14,7±0.1 | 12.0±2.7 | 14.8±0.1 |
| MCHC | 310-347 | g/L | 272.4±3.4 | 224.8±49.8 | 272.5±1.8 |
| CHCM | 307-340 | g/L | 249.2±4.6 | 255.8±3.8 | 247.6±1.3 |
| CH | 13.8-18.4 | pg | 13.4±0.1 | 13.6±0.1 | 13.5±0.0 |
| RDW | 11.7-15.1 | % | 12,9±0.3 | 12.9±0.2 | 12.9±0.1 |
| HDW | 18-26 | g/L | 18,2±0.5 | 18.2±0.3 | 18.4±0.2 |
| PLT | 766-1657 | X10⁹/L | 1155,6±86.4 | 986.0±47.5 | 1106.5±63.9 |
| MPV | 5.0-8.0 | fL | 7,2±0.3 | 7.0±0.3 | 7.5±0.2 |
| %NEUT | 6.8-31.1 | % | 13.9±1.7 | 11.0±2.3 | 12.8±1.5 |
| %LYMPH | 60.2-95.0 | % | 74.3±4.1 | 82.8±2.0 | 80.6±1.3 |
| %MONO | 0-4.3 | % | 2.4±0.6 | 1.3±0.2 | 1.4±0.3 |
| %EOS | 0.2-5.9 | % | 6.9±2.5 | 3.3±0.5 | 3.0±0.6 |
| %BASO | 0-1.0 | % | 0.6±0.2 | 0.3±0.1 | 0.4±0.1 |
| %LUC | 0-3.2 | % | 2.3±0.9 | 1.4±0.5 | 2.0±0.6 |
| #NEUT | 0.5-2.0 | X10⁹/L | 0.6±0.1 | 0.5±0.1 | 0.4±0.1 |
| #LYMPH | 3.8-8.9 | X10⁹/L | 3.6±1.0 | 4.4±0.6 | 3.0±0.6 |
| #MONO | 0-0.3 | X10⁹/L | 0.1±0.0 | 0.1±0.0 | 0.1±0.0 |
| #EOS | 0-0.4 | X10⁹/L | 0.3±0.1 | 0.2±0.0 | 0.1±0.0 |
| #BASO | 0-0.1 | X10⁹/L | 0.1±0.0 | 0.0±0.0 | 0.0±0.0 |
| #LUC | 0-0.3 | X10⁹/L | 0.1±0.0 | 0.1±0.0 | 0.1±0.0 |

| **Table 1** | | | **Day 3** (AM±SE) | | |
|---|---|---|---|---|---|
| TEST | NORMALS | UNITS | Ctrl | p21-hIL-12ORT | p21-hIL-12ORT+EP |
| WBC | 3.2-12.7 | X10⁹/L | 5.2±0.3 | 4.9±0.7 | 4.7±0.6 |
| RBC | 7.0-10.1 | X10¹²/L | 9.8±0.7 | 8.9±1.0 | 9.1±1.2 |
| HGB | 118-149 | g/L | 145.3±11.3 | 131.4±15.3 | 132.4±17.5 |
| HCT | 36.7-46.8 | L/L | 53.0±4.6 | 47.8±5.6 | 48.8±6.9 |
| MCV | 42.2-59.2 | fL | 53.8±0.9 | 53.5±0.8 | 53.5±0.9 |
| MCH | 13.8-18.4 | pg | 14.8±0.1 | 14.7±0.2 | 14.5±0.2 |
| MCHC | 310-347 | g/L | 274.3±3.8 | 274.4±0.8 | 270.8±3.3 |
| CHCM | 307-340 | g/L | 250.0±5.2 | 252.4±3.2 | 248.6±4.0 |
| CH | 13.8-18.4 | pg | 13.4±0.1 | 13.4±0.1 | 13.3±0.1 |
| RDW | 11.7-15.1 | % | 13.0±0.3 | 13.4±0.2 | 13.3±0.5 |
| HDW | 18-26 | g/L | 18.6±0.0 | 19.3±0.4 | 19.8±0.8 |
| PLT | 766-1657 | X10⁹/L | 1055.8±52.2 | 1017.8±66.0 | 1319.8±84.7 |
| MPV | 5.0-8.0 | fL | 6.9±0.4 | 6.6±0.4 | 7.2±0.3 |
| %NEUT | 6.8-31.1 | % | 10.9±2.2 | 11.1±1.2 | 8.9±1.2 |
| %LYMPH | 60.2-95.0 | % | 82.2±2.7 | 81.7±2.0 | 86.3±1.7 |
| %MONO | 0-4.3 | % | 1.7±0.3 | 1.6±0.3 | 0.9±0.2 |
| %EOS | 0.2-5.9 | % | 4.0±1.1 | 4.5±1.2 | 2.6±0.6 |
| %BASO | 0-1.0 | % | 0.3±0.1 | 0.2±0.1 | 0.2±0.1 |
| %LUC | 0-3.2 | % | 0.9±0.2 | 0.9±0.2 | 0.7±0.1 |
| #NEUT | 0.5-2.0 | X10⁹/L | 0.6±0.1 | 0.5±0.1 | 0.4±0.1 |
| #LYMPH | 3.8-8.9 | X10⁹/L | 4.3±0.3 | 4.0±0.6 | 4.1±0.5 |
| #MONO | 0-0.3 | X10⁹/L | 0.1±0.0 | 0.1±0.0 | 0.1±0.0 |
| #EOS | 0-0.4 | X10⁹/L | 0.2±0.1 | 0.2±0.1 | 0.1±0.0 |
| #BASO | 0-0.1 | X10⁹/L | 0.0±0.0 | 0.0±0.0 | 0.0±0.0 |
| #LUC | 0-0.3 | X10⁹/L | 0.0±0.0 | 0,0±0.0 | 0.0±0.0 |

| **Table 1** | | | **Day 6** (AM±SE) | | |
|---|---|---|---|---|---|
| TEST | NORMALS | UNITS | Ctrl | p21-hIL-12ORT | p21-hIL-12ORT+EP |
| WBC | 3.2-12.7 | X10⁹/L | 7.7±0.7 | 7.0±0.2 | 6.5±0.7 |
| RBC | 7.0-10.1 | X10¹²/L | 6.3±1.3 | 8.8±1.2 | 9.7±0.5 |
| HGB | 118-149 | g/L | 93.3±18.4 | 128.8±18.1 | 140.2±8.1 |
| HCT | 36.7-46.8 | L/L | 35.3±5.6 | 46.1±5.8 | 51.6±3.1 |
| MCV | 42.2-59.2 | fL | 55.5±2.8 | 53.3±1.6 | 54.5±0.7 |
| MCH | 13.8-18.4 | pg | 14.9±0.3 | 14.6±0.1 | 14.8±0.1 |
| MCHC | 310-347 | g/L | 269.8±8.8 | 275.3±9.3 | 269.2±4.3 |
| CHCM | 307-340 | g/L | 249.0±10.3 | 255.6±8.3 | 246.2±3.1 |
| CH | 13.8-18.4 | pg | 13.6±0.2 | 13.5±0.1 | 13.4±0.1 |
| RDW | 11.7-15.1 | % | 15.8±1.8 | 14.0±1.3 | 14.9±1.1 |
| HDW | 18-26 | g/L | 24.0±3.7 | 19.3±0.8 | 19.8±0.9 |
| PLT | 766-1657 | X10⁹/L | 995.3±69.4 | 1126.0±54.0 | 1284.8±43.5 |
| MPV | 5.0-8.0 | fL | 7.2±0.6 | 7.2±0.4 | 7.5±0.4 |
| %NEUT | 6.8-31.1 | % | 14.5±1.7 | 11.7±1.4 | 10.1±1.1 |
| %LYMPH | 60.2-95.0 | % | 79.5±1.6 | 81.2±2.2 | 83.4±1.7 |
| %MONO | 0-4.3 | % | 2.2±0.4 | 2.2±0.2 | 2.0±0.3 |
| %EOS | 0.2-5.9 | % | 1.8±0.2 | 3.5±1.1 | 3.3±0.8 |
| %BASO | 0-1.0 | % | 0.2±0.0 | 0.2±0.0 | 0.3±0.0 |
| %LUC | 0-3.2 | % | 1.6±0.1 | 1.3±0.1 | 0.9±0.1 |
| #NEUT | 0.5-2.0 | X10⁹/L | 1.1±0.2 | 0.8±0.1 | 0.6±0.0 |
| #LYMPH | 3.8-8.9 | X10⁹/L | 6.1±0.5 | 5.7±0.3 | 5.4±0.7 |
| #MONO | 0-0.3 | X10⁹/L | 0.2±0.0 | 0.2±0.0 | 0.1±0.0 |
| #EOS | 0-0.4 | X10⁹/L | 0.1±0.0 | 0.2±0.1 | 0.2±0.0 |
| #BASO | 0-0.1 | X10⁹/L | 0.0±0.0 | 0.0±0.0 | 0.0±0.0 |
| #LUC | 0-0.3 | X10⁹/L | 0.0±0.0 | 0.1±0.0 | 0.1±0.0 |

| **Table 1** | | | **Day 8** (AM±SE) | | |
|---|---|---|---|---|---|
| TEST | NORMALS | UNITS | Ctrl | p21-hIL-12ORT | p21-hIL-12ORT+EP |
| WBC | 3.2-12.7 | X10⁹/L | 6.1±1.6 | 6.4±1.4 | 6.2±0.8 |
| RBC | 7.0-10.1 | X10¹²/L | 4.1 ±1.0* | 5.4±1.7 | 8.1±1.2 |
| HGB | 118-149 | g/L | 59.6±13.4** | 76.5±26.4+ | 121.0±17.6 |
| HCT | 36.7-46.8 | L/L | 23.2±4.8++ | 31.8±8.7 | 46.3±5.1 |
| MCV | 42.2-59.2 | fL | 57.5±3.2 | 62.7±5.0 | 60.5±4.2 |
| MCH | 13.8-18.4 | pg | 14.6±0.5 | 13.7±1.5 | 14.9±0.2 |
| MCHC | 310-347 | g/L | 256.4±8.7 | 221.0±26.1 | 252.1±14.0 |
| CHCM | 307-340 | g/L | 238.0±11.1 | 231.0±11.3 | 230.8±12.2 |
| CH | 13.8-18.4 | pg | 13.5±0.3 | 14.0±0.4 | 13.6±0.2 |
| RDW | 11.7-15.1 | % | 17.0±2.7 | 20.0±2.6 | 16.7±1.8 |
| HDW | 18-26 | g/L | 23.2±2.1 | 27.3±3.8 | 24.0±2.7 |
| PLT | 766-1657 | X10⁹/L | 1001.4±118.8 | 923.8±87.7 | 1067.4±101.8 |
| MPV | 5.0-8.0 | fL | 7.4±0.4 | 7.6±0.6 | 8.2±0.8 |
| %NEUT | 6.8-31.1 | % | 27.1±6.7 | 19.7±5.4 | 9.5±0.9 |
| %LYMPH | 60.2-95.0 | % | 67.7±6.9 | 74.7±5.3 | 83.0±2.3 |
| %MONO | 0-4.3 | % | 2.1±0.2 | 1.7±0.3 | 2.2±0.5 |
| %EOS | 0.2-5.9 | % | 1.7±0.4 | 2.0±0.7 | 4.3±1.4 |
| %BASO | 0-1.0 | % | 0.2±0.1 | 0.2±0.1 | 0.2±0.0 |
| %LUC | 0-3.2 | % | 1.3±0.2 | 1.8±0.9 | 0.8±0.1 |
| #NEUT | 0.5-2.0 | X10⁹/L | 1.3±0.2 | 1.2±0.4 | 0.6±0.1 |
| #LYMPH | 3.8-8.9 | X10⁹/L | 4.6±1.4 | 5.0±1.4 | 5.2±0.8 |
| #MONO | 0-0.3 | X10⁹/L | 0.1±0.0 | 0.1±0.0 | 0.1±0.0 |
| #EOS | 0-0.4 | X10⁹/L | 0.1±0.0 | 0.1±0.0 | 0.2±0.1 |
| #BASO | 0-0.1 | X10⁹/L | 0.1±0.0 | 0.1±0.0 | 0.0±0.0 |
| #LUC | 0-0.3 | X10⁹/L | 0.1±0.0 | 0.1±0.0 | 0.1±0.0 |

| **Table 2** | | | **Day 1** (AM±SE) | | |
|---|---|---|---|---|---|
| TEST | NORMALS | UNITS | Ctrl | p21-hIL-12ORT | p21-hIL-12ORT+EP |
| Creatinine | 0,2-0,9 | mg/dl | 0.4±0.0 | 0.4±0.0 | 0.4±0.0 |
| TP2 | 3,5-7,2 | g/dl | 5.3±0.0 | 5.1±0.1 | 5.4±0.0 |
| Albumin | 2,5-3 | g/dl | 3.6±0.0 | 3.4±0.2 | 3.6±0.1 |

| **Table 2** | | | **Day 6** (AM±SE) | | |
|---|---|---|---|---|---|
| TEST | NORMALS | UNITS | Ctrl | p21-hIL-12ORT | p21-hIL-12ORT+EP |
| Creatinine | 0,2-0,9 | mg/dl | 0.4±0.0 | 0.4±0.0 | 0.4±0.0 |
| TP2 | 3,5-7,2 | g/dl | 4.5±0.2 | 5.0±0.3 | 5.2±0.1 |
| Albumin | 2,5-3 | g/dl | 3.0±0.1 | 3.4±0.2 | 3.5±0.1 |

| **Table 2** | | | **Day 8** (AM±SE) | | |
|---|---|---|---|---|---|
| TEST | NORMALS | UNITS | Ctrl | p21-hIL-12ORT | p21-hIL-12ORT+EP |
| Creatinine | 0,2-0,9 | mg/dl | 0.4±0.0 | 0.4±0.0 | 0.4±0.0 |
| TP2 | 3,5-7,2 | g/dl | 4.7±0.2 | 4.2±0.3 | 5.0±0.3 |
| Albumin | 2,5-3 | g/dl | 3.2±0.3 | 3.0±0.2 | u |

### References

[1] Francken AB, Bastiaannet E, Hoekstra HJ. Follow-up in patients with localised primary cutaneous melanoma. The Lancet Oncology. 2005 Aug;6(8):608-21. PubMed PMID: 16054572.
[2] Trinchieri G. Interleukin-12 - A Proinflammatory Cytokine with Immunoregulatory Functions That Bridge Innate Resistance and Antigen-Specific Adaptive Immunity. Annu Rev Immunol. 1995 1995;13:251-76.
[3] Trinchieri G. Interleukin-12 and the regulation of innate resistance and adaptive immunity. NatRevlmmunol. 2003 2/2003;3(2):133-46.
[4] Voest EE, Kenyon BB, Oreilly MS, Truitt G, Damato RJ, Folkman J. Inhibition of Angiogenesis In-Vivo by Interleukin-12. J Natl Cancer I. 1995 4/19/1995;87(8):581-6.
[5] Ogawa M, Yu WG, Umehara K, Iwasaki M, Wijesuriya R, Tsujimura T, et al. Multiple roles of interferon-gamma in the mediation of interleukin 12-induced tumor regression. Cancer Res. 1998 6/1/1998;58(11):2426-32.
[6] Boehm T, Folkman J, Browder T, O'Reilly MS. Antiangiogenic therapy of experimental cancer does not induce acquired drug resistance. Nature. 1997 1997;390(6658):404-7.
[7] Brunda MJ, Luistro L, Warrier RR, Wright RB, Hubbard BR, Murphy M, et al. Antitumor and Antimetastatic Activity of Interleukin-12 Against Murine Tumors. J Exp Med. 1993 10/1/1993;178(4):1223-30.
[8] Cohen J. IL-12 deaths: explanation and a puzzle. Science. 1995 Nov 10;270(5238):908. PubMed PMID: 7481785.
[9] Leonard JP, Sherman ML, Fisher GL, Buchanan LJ, Larsen G, Atkins MB, et al. Effects of single-dose interleukin-12 exposure on interleukin-12-associated toxicity and interferon-gamma production. Blood. 1997 Oct 1;90(7):2541-8. PubMed PMID: 9326219.
[10] Heller LC, Heller R. Electroporation gene therapy preclinical and clinical trials for melanoma. Current gene therapy. 2010 Aug;10(4):312-7. PubMed PMID: 20557286.
[11] Cemazar M, Jarm T, Sersa G. Cancer Electrogene Therapy with Interleukin-12. Current gene therapy. 2010 Aug;10(4):300-11. PubMed PMID: WOS:000280525500006. English.
[12] Daud Al, DeConti RC, Andrews S, Urbas P, Riker Al, Sondak VK, et al. Phase I Trial of Interleukin-12 Plasmid Electroporation in Patients With Metastatic Melanoma. J Clin Oncol. 2008 12/20/2008;26(36):5896-903.
[13] Heller KN, Pavlick AC, Hodi FS, Thompson JA, Margolin KA, Lawrence DP, et al. Safety and survival analysis of ipilimumab therapy in patients with stable asymptomatic brain metastases. J Clin Oncol. 2011 May 20;29(15). PubMed PMID: WOS:000208880302439.
[14] Lucas ML, Heller L, Coppola D, Heller R. IL-12 plasmid delivery by in vivo electroporation for the successful treatment of established subcutaneous B16.F10 melanoma. Mol Ther. 2002 Jun;5(6):668-75. PubMed PMID: WOS:000176082700006.
[15] Daud Al, DeConti RC, Andrews S, Urbas P, Riker Al, Sondak VK, et al. Phase I trial of interleukin-12 plasmid electroporation in patients with metastatic melanoma. Journal of clinical oncology : official journal of the American Society of Clinical Oncology. 2008 Dec 20;26(36):5896-903. PubMed PMID: 19029422. Pubmed Central PMCID: 2645111.
[16] Pavlin M, Pucihar G, Kanduser M. The role of electrically stimulated endocytosis in gene electrotransfer. Bioelectrochemistry. 2012 Feb;83:38-45. PubMed PMID: WOS:000297962500007.
[17] Seetharam S, Staba MJ, Schumm LP, Schreiber K, Schreiber H, Kufe DW, et al. Enhanced eradication of local and distant tumors by genetically produced interleukin-12 and radiation. Int J Oncol. 1999 10/1999;15(4):769-73.
[18] Alatrash G, Hutson TE, Molto L, Richmond A, Nemec C, Mekhail T, et al. Clinical and immunologic effects of subcutaneously administered interleukin-12 and interferon alfa-2b: phase I trial of patients with metastatic renal cell carcinoma or malignant melanoma. JClinOncol. 2004 7/15/2004;22(14):2891-900.
[19] Tevz G, Kranjc S, Cemazar M, Kamensek U, Coer A, Krzan M, et al. Controlled systemic release of interleukin-12 after gene electrotransfer to muscle for cancer gene therapy alone or in combination with ionizing radiation in murine sarcomas. J Gene Med. 2009 12/2009;11(12):1125-37.
[20] Sedlar A, Kranjc S, Dolinsek T, Cemazar M, Coer A, Sersa G. Radiosensitizing effect of intratumoral interleukin-12 gene electrotransfer in murine sarcoma. BMC cancer. 2013;13:38. PubMed PMID: 23360213. Pubmed Central PMCID: 3562515.
[21] Jinushi M, Tahara H. Cytokine gene-mediated immunotherapy: current status and future perspectives. Cancer Sci. 2009 Aug;100(8):1389-96. PubMed PMID: 19459853.
[22] Mehlen P, Puisieux A. Metastasis: a question of life or death. Nature reviews Cancer. 2006 Jun;6(6):449-58. PubMed PMID: 16723991.
[23] Young JL, Dean DA. Electroporation-mediated gene delivery. Advances in genetics. 2015;89:49-88. PubMed PMID: 25620008.
[24] Spanggaard I, Snoj M, Cavalcanti A, Bouquet C, Sersa G, Robert C, et al. Gene electrotransfer of plasmid antiangiogenic metargidin peptide (AMEP) in disseminated melanoma: safety and efficacy results of a phase I first-in-man study. Hum Gene Ther Clin Dev. 2013 Sep;24(3):99-107. PubMed PMID: 23980876.
[25] Chiarella P, Fazio VM, Signori E. Electroporation in DNA Vaccination Protocols Against Cancer. Current Drug Metabolism. 2013 Mar;14(3):291-9. PubMed PMID: WOS:000317118400004.
[26] Gill DR, Pringle IA, Hyde SC. Progress and Prospects: The design and production of plasmid vectors. Gene Ther. 2009 Feb;16(2):165-71. PubMed PMID: WOS:000263892800001.
[27] Kamensek S, Podlesek Z, Gillor O, Zgur-Bertok D. Genes regulated by the Escherichia coli SOS repressor LexA exhibit heterogenous expression. Bmc Microbiology. 2010 Nov 11;10. PubMed PMID: WOS:000284759300001.
[28] Harada K, Ogden GR. An overview of the cell cycle arrest protein, p21(WAF1). Oral Oncology. 2000 1/2000;36(1):3-7.
[29] Eldeiry WS, Tokino T, Velculescu VE, Levy DB, Parsons R, Trent JM, et al. Waf1, A Potential Mediator of P53 Tumor Suppression. Cell. 1993 11/19/1993;75(4):817-25.
[30] Robson T, Hirst DG. Transcriptional targeting in cancer gene therapy. Journal of Biomedicine and Biotechnology. 2003 4/21/2003;2003(2):110-37.
[31] Robson T, Worthington J, McKeown SR, Hirst DG. Radiogenic therapy: Novel approaches for enhancing tumor radiosensitivity. Technol Cancer Res T. 2005 8/2005;4(4):343-61.
[32] Kamensek U, Sersa G, Cemazar M. Evaluation of p21 promoter for interleukin 12 radiation induced transcriptional targeting in a mouse tumor model. Molecular Cancer. 2013 Nov 12;12. PubMed PMID: WOS:000329124900002.
[33] Solensky R. Hypersensitivity reactions to beta-lactam antibiotics. Clin Rev Allerg Immu. 2003 Jun;24(3):201-19. PubMed PMID: WOS:000182633400002.
[34] McMahon JM, Wells DJ. Electroporation for gene transfer to skeletal muscles - Current status. Biodrugs. 2004;18(3):155-65. PubMed PMID: WOS:000222165800002. English.
[35] Krieg AM. CpG motifs in bacterial DNA and their immune effects. Annu Rev Immunol. 2002;20:709-60. PubMed PMID: WOS:000175012600023.
[36] Needleman SB, Wunsch CD. A general method applicable to the search for similarities in the amino acid sequence of two proteins. J Mol Biol. 1970 Mar;48(3):443-53.

## Claims

1. An expression plasmid comprising a polynucleotide encoding an IL-12 (Interleukin 12) polypeptide and operably linked thereto a CDKN1A promoter, wherein said expression plasmid does not comprise a selectable marker gene.

2. The expression plasmid of claim 1, wherein the IL-12 polypeptide is the human IL-12 polypeptide.

3. The expression plasmid of claims 1 and 2, wherein the CDKN1A promoter is the human CDKN1A promoter.

4. The expression plasmid of any one of claims 1 to 3, wherein the expression plasmid comprises a pUC origin of replication.

5. The expression plasmid of any one of claims 1 to 4, wherein the expression plasmid comprises a lac operator sequence.

6. The expression plasmid of any one of claims 1 to 5, wherein the expression plasmid has a sequence as shown in SEQ ID NO:1.

7. The expression plasmid of any one of claims 1 to 6 for use in the treatment of cancer.

8. The expression plasmid for use of claim 7, wherein the cancer is sarcoma, carcinoma, lymphoma, melanoma or haemangioma, in particular wherein the cancer is melanoma.

9. The expression plasmid for use of claim 7 or 8, wherein the expression plasmid is administered intratumorally or peritumorally.

10. The expression plasmid for use of any one of claims to 7 to 9, wherein the expression plasmid is transfected into cells of the patient by electroporation.

11. A host cell comprising the expression plasmid of any one of claims 1 to 6.

12. The host cell of claim 11, wherein the host cell further comprises an essential gene operably linked the *lac* operator/promoter *(IacO*/*P).*

13. The host cell of claims 11 or 12, wherein the host cell is an *E. coli* cell.

14. A method for producing the expression plasmid of any one of claims 1 to 6, said method comprising:
a) cultivating the host cell of any one of claims 10 to 13, and
b) extracting the expression plasmid from said host cell.

15. A pharmaceutical composition comprising the expression plasmid of any one of claims 1 to 6.
